# FASCICULE DE BREVET EUROPEEN

(11) **EP 0 952 794 B1**
(45) Date de publication et mention de la délivrance du brevet: **12.06.2002**
(21) Numéro de dépôt: 97947104.2
(22) Date de dépôt: 20.11.1997
(51) Int. Cl.: A61F 2/06

(54) **ENDOPROTHESE MODULAIRE ET EXPANSIBLE POUR LE RESEAU ARTERIEL**
MODULARE, EXPANDIERBARE ENDOPROTHESE FUR DAS ARTERIENSYSTEM
MODULAR AND EXPANDABLE ENDOPROSTHESIS FOR THE ARTERIAL NETWORK

(30) Priorité: 22.11.1996 FR 9614286
(43) Date de publication de la demande: 03.11.1999
(73) Titulaire: Marcade, Jean-Paul, 17000 La Rochelle (FR)
(72) Inventeur: Marcade, Jean-Paul, 17000 La Rochelle (FR)
(74) Mandataire: Burtin, Jean-François
(86) Numéro de dépôt international: FR9702094
(87) Numéro de publication internationale: WO9822043

(56) Documents cités:
- EP-A- 0 722 701
- WO-A-93/05730

## Description

La présente invention concerne le domaine de la chirurgie et plus particulièrement celui des dispositifs utilisés en chirurgie cardio-vasculaire.

Elle a plus spécifiquement pour objet une endoprothèse modulaire et expansible pour la reconstruction du réseau artériel de l'organisme humain ou animal.

Il y a deux grands types de pathologie artérielle : la pathologie obstructive, qui consiste en un épaississement des parois artérielles par des dépôts, aboutissant au rétrécissement puis à l'occlusion de l'artère et entraînant une insuffisance circulatoire ou ischémie et, la pathologie anévrismale. L'anévrisme est une dilatation anormale de l'artère avec perte du parallélisme des parois ; cette dilatation fusiforme ou sacciforme, fragilise l'artère qui un jour peut se rompre sous l'action de la pression artérielle entraînant une hémorragie interne cataclysmique et en général fatale.

Pour réparer ou reconstruire une artère pathologique, deux techniques sont possibles. La première est la technique chirurgicale conventionnelle qui aborde l'artère pathologique à travers une plaie opératoire et une dissection, en général en vue d'un pontage c'est-à-dire d'un remplacement du segment défectueux par suture directe d'un nouveau conduit reliant les parties saines d'amont et d'aval.
La seconde est la technique endoluminale ou endovasculaire qui vise à traiter l'artère malade de l'intérieur même de la lumière, par cathétérisme. Cette technique, qui évite une plaie opératoire est un progrès récent. Elle est de plus en plus utilisée au fil des améliorations technologiques et permet notamment l'introduction de prothèses ou nouveaux conduits synthétiques à l'intérieur même de la lumière artérielle pathologique pour rétablir des conditions anatomiques et hémodynamiques correctes.

Le segment artériel à reconstruire par voie endoluminale peut être un segment rectiligne ou peut concerner une zone de bifucartion. La bifurcation artérielle la plus fréquemment atteinte en pathologie humaine est la bifurcation aortique.

L'aorte est le tronc artériel principal du corps. Il prend naissance au niveau du ventricule gauche du coeur, traverse la cage thoracique, descend dans l'abdomen jusqu'au niveau du petit bassin où il se bifurque en deux artères iliaques destinées à la vascularisation de chacun des membres inférieurs. C'est ce segment artériel appelé aorte sous-rénale et bifurcation aortique, qui constitue le principal emplacement susceptible d'être endommagé, et qui suscite un besoin de réparation (fréquence des anévrismes de l'aorte abdominale et des anévrismes aorto-iliaques).

La présente invention concerne une prothèse destinée à la reconstruction de toute bifurcation artérielle et notamment à la reconstruction de l'aorte abdominale sous-rénale et de la bifurcation aortique c'est-à-dire de la portion terminale de l'aorte en aval du départ des artères rénales et de sa bifurcation en iliaque droite et iliaque gauche.
La prothèse de la présente invention est destinée plus particulièrement au traitement non chirurgical des anévrismes aorto-iliaques par la procédure endoluminale. A ce titre et dans cette application, cette prothèse pourra être dénommée « endoprothèse aorto-iliaque ».

Les prothèses artérielles actuellement validées et largement utilisées sont des conduits synthétiques en matériau complètement inerte, biocompatible comme le polytétrafluoroéthylène (PTFE) ou les polyesters (DACRON tricoté ou tissé).
Lorsque les prothèses du type endoluminal sont introduites à l'intérieur d'un vaisseau par cathétérisme, leur suture à la paroi artérielle normale n'est pas possible, et la fixation de la prothèse à la paroi artérielle normale doit donc être assurée différemment de la technique chirurgicale classique qui utilise du fil et une aiguille.
Cette fixation est assurée en plaquant le tissu synthétique contre la paroi artérielle par l'intermédiaire d'une pièce métallique, endoprothèse métallique ou stent, à chaque extrémité. Ces pièces métalliques se présentent sous forme de treillis cylindrique, soit auto-expansible (alliage à mémoire thermique de forme comme le Nitinol), soit expansible à l'aide d'un ballonnet.
Dans certains cas, le tissu synthétique de la prothèse est soutenu par une armature métallique ou une cage métallique sur toute sa hauteur pour favoriser son ouverture et sa stabilité à l'intérieur du vaisseau.
Dans le cas relativement simple de la reconstruction d'un segment artériel rectiligne, on fait appel à des « tubes ». Dans le cas beaucoup plus complexe de la reconstruction d'une bifurcation artérielle comme la bifurcation aortique, il se pose un problème technique difficile, la prothèse déployée à l'intérieur du vaisseau devant reproduire elle-même une bifurcation.

Pour répondre à ce problème de bifurcation, on a déjà proposé une endoprothèse modulaire constituée de deux pièces ou prothèses. La première prothèse reproduit une bifurcation dont on aurait amputé l'une des jambes. Cette première prothèse est introduite par cathétérisme d'une des artères iliaques, elle comporte un corps qui se déploie dans l'aorte et une jambe en continuité qui se déploie dans l'artère iliaque cathétérisée. La deuxième prothèse est un tube ; elle est introduite par l'artère iliaque opposée et vient se loger et s'assembler au corps de la prothèse précédente pour former la deuxième jambe iliaque.
Une telle eudoprothèse est par exemple illustée par la demande de brevet WO-A-95/21592. Cette eudoprothèse présente notamment toute les caractéristiques du préambule de la revendication 1.
Ce mode d'assemblage présente une grande difficulté qui est celle du cathétérisme pour la mise en place de la deuxième jambe iliaque.
En effet, la première prothèse qui se compare à une bifurcation amputée d'un côté, doit être cathétérisée à travers le moignon amputé pour pouvoir greffer à l'intérieur la deuxième prothèse. Or, l'orifice de ce moignon est relativement étroit, il est difficile à trouver et les cathéters ne sont pas facilement guidés par les manoeuvres de l'opérateur.
Cette procédure d'assemblage des deux pièces ou prothèses est souvent longue avant de réussir et parfois elle ne réussit pas.

La présente invention a donc pour but de fournir une endoprothèse qui élimine les inconvénients énumérés ci-dessus.

A cet effet, elle a pour objet une endoprothèse modulaire et expansible pour la reconstruction d'une bifurcation artérielle, constituée d'un corps prothétique principal, de deux branches prothétiques de bifurcation et éventuellement d'un ou de plusieurs étages d'ajustage, caractérisée en ce que le corps prothétique principal est un corps prothétique bifide, en forme de tube ou cylindre unique à ses extrémités proximale et distale dénommées extrémité tubulaire proximale et extrémité tubulaire distale, se dédoublant ou se divisant entre ses extrémités tubulaires proximale et distale en deux conduits séparés et indépendants, dénommés branches ou tubes de bifidité.

Les deux branches prothétiques de bifurcation sont des tubes qui viennent se loger facilement dans chaque branche ou tube de bifidité du corps prothétique principal, aboutissant ainsi à la construction d'une prothèse bifurquée, objet de l'invention.

Les deux branches ou tubes de bifidité du corps prothétique principal présentent une longueur et une position variables par rapport aux extrémités tubulaires uniques proximale et distale dudit corps prothétique principal.
Les deux branches ou tubes de bifidité se situent de préférence dans la partie médiane du corps prothétique principal, mais elles peuvent également se situer plus près de l'extrémité tubulaire distale ou de l'extrémité tubulaire proximale du corps prothétique bifide principal.

La longueur du corps prothétique principal bifide ainsi que la position et la longueur des deux branches ou tubes de bifidité par rapport aux extrémités tubulaires uniques proximale et distale, sont déterminées en fonction de la longueur de l'artère à réparer ainsi que des particularités anatomiques de la lésion à traiter.

De préférence, les extrémités tubulaires du corps prothétique principal bifide ont une section circulaire. Cependant, en raison des problèmes anatomiques rencontrés et de la nécessité d'une jonction parfaitement étanche ou non entre les extrémités de la prothèse et la paroi artérielle en amont et en aval de la zone traitée, les extrémités de la prothèse pourront présenter une section différente.

Le rapport entre le diamètre d'une branche ou tube de bifidité et le diamètre de l'extrémité tubulaire unique proximale ou distale est de préférence égal à 0,5. Cependant, ce rapport peut également être supérieur ou inférieur au rapport exact de 0,5.

Les diamètres respectifs des deux branches ou tubes de bifidité du corps prothétique principal sont identiques ou différents.
Les diamètres respectifs des extrémités tubulaires uniques proximale et distale du corps prothétique bifide principal sont identiques ou différents.
L'endoprothèse modulaire est auto-expansible ou expànsible mécaniquement. En cas de prothèse expansible mécaniquement, par exemple avec un ballonnet, le diamètre des deux tubes ou branches de bifidité et le diamètre des deux extrémités tubulaires proximale et distale varient selon la technique d'expansion et les capacités d'expansion du corps prothétique principal et selon le diamètre de l'artère à laquelle la prothèse doit s'adapter. Le diamètre de l'extrémité tubulaire proximale est calculé de façon à occuper toute la lumière artérielle (plus précisément la lumière aortique), pour une parfaite étanchéité de la jonction prothéto-artérielle (prothéto-aortique) proximale.

Le corps prothétique principal bifide et les deux branches prothétiques tubulaire de bifurcation constituant l'endoprothèse selon l'invention pourront encore être dénommés : corps prothétique artériel principal, et branches prothétiques artérielles de bifurcation.
Dans le cas d'une bifurcation aorto-iliaque, on pourra les dénommer de façon encore plus précise, à savoir : corps prothétique aortique bifide et branches prothétiques iliaques de bifurcation.

Les branches prothétiques tubulaires de bifurcation, iliaque droite et iliaque gauche, sont introduites par cathétérisme à l'intérieur de chaque branche ou tube de bifidité du corps prothétique principal aortique.

Ainsi en pathologie aorto-iliaque, l'endoprothèse selon l'invention conserve le principe d'un assemblage prothétique au niveau du segment aortique mais dans la présente invention, chacune des deux branches prothétiques de bifurcation est disposée séparément à l'intérieur des deux branches ou tubes de bifidité du corps prothétique principal bifide.

Les deux branches prothétiques de bifurcation peuvent être disposées à des niveaux différents dans chacune des deux branches ou tubes de bifidité du corps prothétique principal, en faisant varier le niveau d'ancrage de chaque branche prothétique de bifurcation dans la branche ou tube de bifidité correspondant.
Le corps prothétique bifide principal présente donc l'avantage de permettre une grande facilité de montage ou d'ajustage pour chaque branche prothétique de bifurcation en fonction des conditions anatomiques, le segment bifide du corps prothétique principal le permettant par un effet de coulissement à l'image d'un trombone. L'appréciation des différents niveaux des deux branches prothétique de bifurcation peut comporter des différences sensibles, et la tolérance pourra être de plusieurs centimètres.

Le corps prothétique principal bifide selon la présente invention n'est donc plus une bifurcation amputée de ses deux jambes mais un tube dont la partie médiane est divisée en deux (tube bifide) ce qui laisse à la partie inférieure (extrémité tubulaire distale) un entonnoir prothétique large plus facile à cathétériser que l'orifice d'amputation d'une branche de bifurcation amputée.

L'endoprothèse modulaire et expansible est constituée d'un tissu synthétique étanche ou devenant étanche au contact du sang, stable, inerte, biocompatible soutenu par une armature ou par un treillis métallique.
L'armature métallique ou stent peut être autoexpansible (Nitinol) ou expansée à l'aide de ballonnets de dilatation (acier inoxydable, tantale..).
Le tissu synthétique peut être du polytétrafluoroéthylène (PTFE) ou un polyester (Dacron) ou tout autre matériau biocompatible.
L'endoprothèse peut également être constituée d'un treillis métallique noyé dans un film en matière plastique ou élastomère, extensible et biocompatible, ce film emplissant les mailles du treillis et recouvrant la totalité de la surface du treillis.

Le film est dans ce cas constitué d'un polymère biocompatible tel qu'un PTFE ou un polyuréthanne ou une silicone ou un latex ou autre.

Un ou des ballonnets permettent ou aident le déploiement de l'endoprothèse et notamment l'ouverture des tubes ou branches de bifidité du corps prothétique principal, l'ouverture des extrémités tubulaires proximale et distale du corps prothétique principal, ainsi que l'ouverture des branches prothétiques de bifurcation. Ils permettent également d'assurer une dilatation plus ou moins importante et réglable.

Les ballonnets permettent par exemple dans le cas d'endoprothèse modelable (c'est-à-dire avec armature ou stent non auto-expansible) de surdilater les points « d'ancrage» de l'endoprothèse et permettent notamment d'élargir le diamètre de la partie supérieure de l'extrémité tubulaire proximale du corps prothétique principal, ainsi que certaines parties des branches prothétiques de bifurcation, d'une manière parfaitement adapté à l'ajustement des prothèses.

Le caractère modulaire ou modularité de l'endoprothèse permet d'ajouter un ou plusieurs étages d'ajustage sur la partie supérieure de l'extrémité tubulaire proximale du corps prothétique bifide principal pour que l'ensemble s'adapte parfaitement au niveau recherché dans l'artère, par exemple dans le cas d'un anévrisme de l'aorte abdominale au collet supérieur de l'anévrisme. Cette modularité permet également des rattrapages secondaires par exemple pour parfaire une étancheité initialement imparfaite ou devenue imparfaite, à l'extrémité tubulaire proximale de l'endoprothèse.

Le corps prothétique principal bifide endo-aortique selon l'invention présente de nombreux avantages.
Ainsi, le cathétérisme de la pièce aortique ainsi configurée, depuis chaque artère iliaque, est grandement facilité. L'extrémité inférieure du corps prothétique aortique principal est large et se comporte dans la lumière aortique comme un entonnoir facile à cathétériser depuis chaque artère iliaque puisqu'il occupe toute la lumière aortique. La procédure de cathétérisme pour l'implantation des branches prothétiques iliaques devient simple et rapide.

La possibilité d'ajuster chaque branche prothétique de bifurcation sur une longueur variable à l'intérieur de chaque branche ou tube de bifidité du corps prothétique principal facilite l'adéquation de la longueur totale du montage à l'anatomie du sujet, les mesures pré-opératoires sur imagerie radiographique n'étant pas toujours très précises et la longueur des matériels préfabriqués n'étant pas toujours parfaitement adaptée.
L'ensemble de l'endoprothèse ainsi assemblée gagne aussi en stabilité et en adaptabilité, la division du tube aortique s'adaptant mieux à nombre de situations anatomiques où la lumière aortique résiduelle n'est pas rectiligne mais oblige le matériel à suivre certaines courbes ou tortuosités sans plicature.

Des exemples de réalisation de l'invention vont à présent être décrits en regard des dessins annexés.

Les figures 1 à 21 sont des exemples d'application dans le cadre d'une endoprothèse modulaire à expanser avec des ballonnets, mais que l'on peut envisager aussi avec une endoprothèse auto-expansible.
La figure 1 représente schématiquement le corps prothétique bifide principal selon l'invention, à l'état rétracté.
La figure 2 représente schématiquement le même corps prothétique principal, après dilatation.
La figure 3 représente le corps prothétique principal bifide muni des deux branches prothétiques de bifurcation dans chacun de ses deux tubes ou branches de bifidité.
La figure 4 illustre la possibilité d'ajouter un étage en amont du corps prothétique principal bifide.
La figure 5 illustre la même possibilité que la figure 4, et montre le corps prothétique principal bifide muni de deux branches prothétiques de bifurcation.
Les figures 6 et 7 illustrent l'ouverture des branches de bifidité du corps prothétique principal bifide à l'aide de ballonnets.
Les figures 8 à 10 mettent en évidence l'ouverture de la partie supérieure de l'extrémité tubulaire proximale du corps prothétique principal bifide à l'aide d'un ballonnet.
Les figures 11 et 12 illustrent l'ascension et l'ouverture des branches prothétiques de bifurcation dans le corps prothétique principal bifide.
Les figures 13 à 16 mettent en évidence la possibilité de surdilater les points d'ancrage de l'endoprothèse.
Les figures 17 à 19 illustrent le montage d'un étage d'ajustage supplémentaire en procédant à une dilatation par ballonnet.
Les figures 20 et 21 montrent l'aspect de l'endoprothèse une fois mise en place, et sa disposition au niveau d'un anévrisme de l'aorte abdominale.

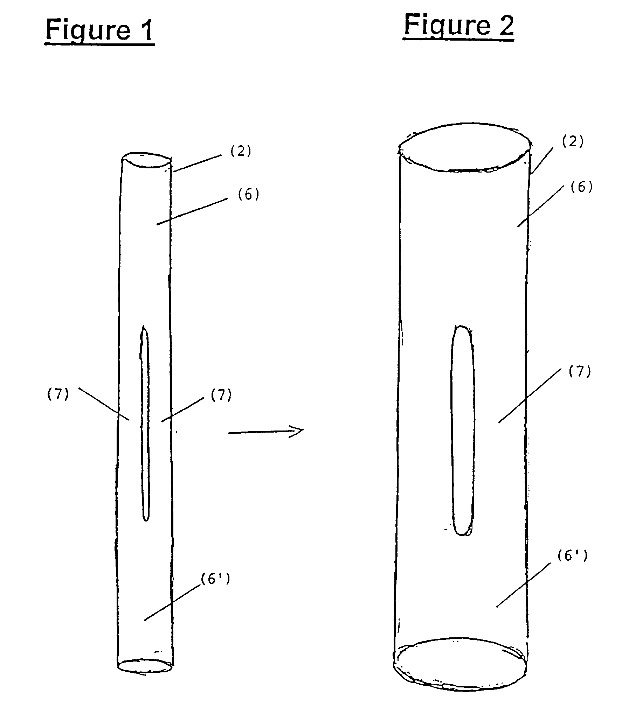

Dans un mode d'exécution actuellement préféré, les figures 1 et 2 représentent le corps prothétique principal bifide (2) selon l'invention, constitué d'une extrémité tubulaire proximale (6) se divisant en deux tubes ou branches de bifidité (7), qui reconfluent ensuite en une extrémité tubulaire unique distale (6').
Le corps prothétique bifide artériel principal (2), en tube bifide à sa partie médiane, pour la reconstruction endo-luminale aorto-iliaque, est représenté à l'état rétracté à la figure 1 et à l'état dilaté à la figure 2.

Le corps prothétique bifide principal (2) présente une longueur comprise entre 1 et 20 cm, et de préférence entre 8 et 12 cm.
Le diamètre des extrémités tubulaires uniques proximale (6) et distale (6') du corps prothétique principal (2) est compris entre 2 et 35 mm, et de préférence entre 12 et 25 mm, suivant qu'il se trouve dans un état rétracté ou dilaté, c'est-à-dire selon le degré d'expansion.
Les deux tubes ou branches de bifidité (7) du corps prothétique principal bifide (2) présentent une longueur comprise entre 0,1 et 18 cm, et de préférence entre 1 et 5 cm, et un diamètre compris entre 0,1 et 20 mm, et de préférence entre 7 et 12 mm, selon le degré d'expansion.

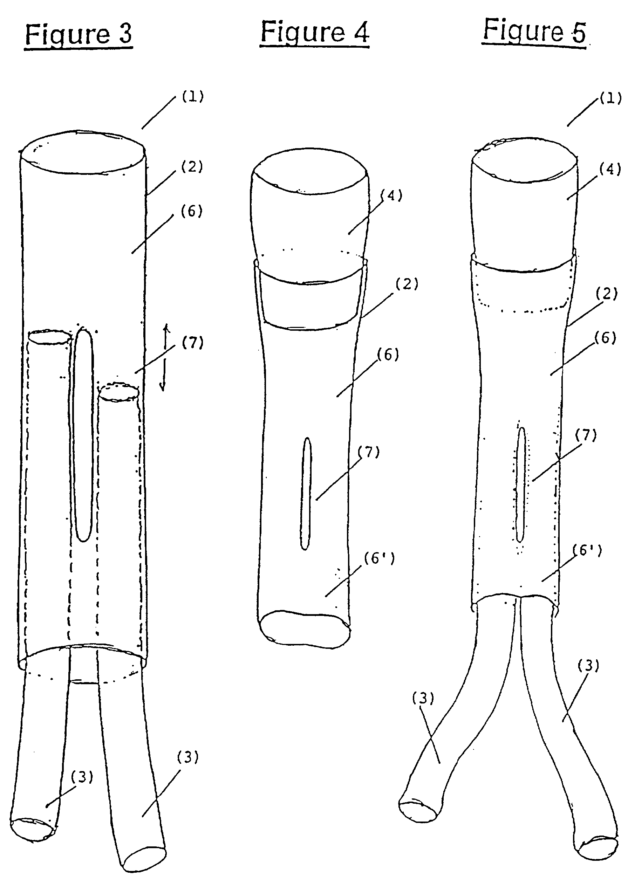

L'endoprothèse (1) représentée à la figure 3 est constituée d'un corps prothétique principal bifide (2), et de deux branches prothétiques de bifurcation (3) disposées séparément à l'intérieur des deux branches ou tubes de bifidité (7) du corps prothétique principal.

La figure 3 illustre la possibilité d'ajuster chaque branche prothétique de bifurcation (3) sur une longueur variable à l'intérieur de chaque branche ou tube de bifidité (7) du corps prothétique principal (2) afin de permettre une meilleure adaptation aux conditions anatomiques particulières de la réparation.
Ainsi, une des deux branches prothétiques de bifurcation (3) peut être disposée au même niveau ou au contraire à un niveau différent de celui de l'autre branche prothétique de bifurcation (3) à l'intérieur des branches ou tubes de bifidité (7).

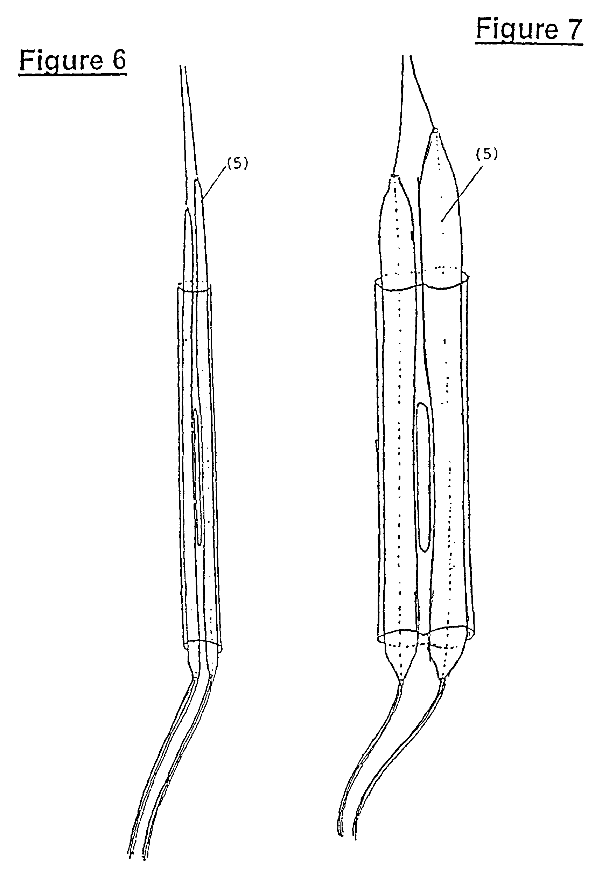

Dans un mode d'exécution particulier de la présente invention, représenté dans les figures 6 à 19, l'endoprothèse modulaire (1) est expansée à l'aide de ballonnets (5). Les figures 6 et 7 illustrent l'ouverture des branches ou tubes de bifidité (7) du corps prothétique principal bifide (2) à l'aide de ballonnets (5).

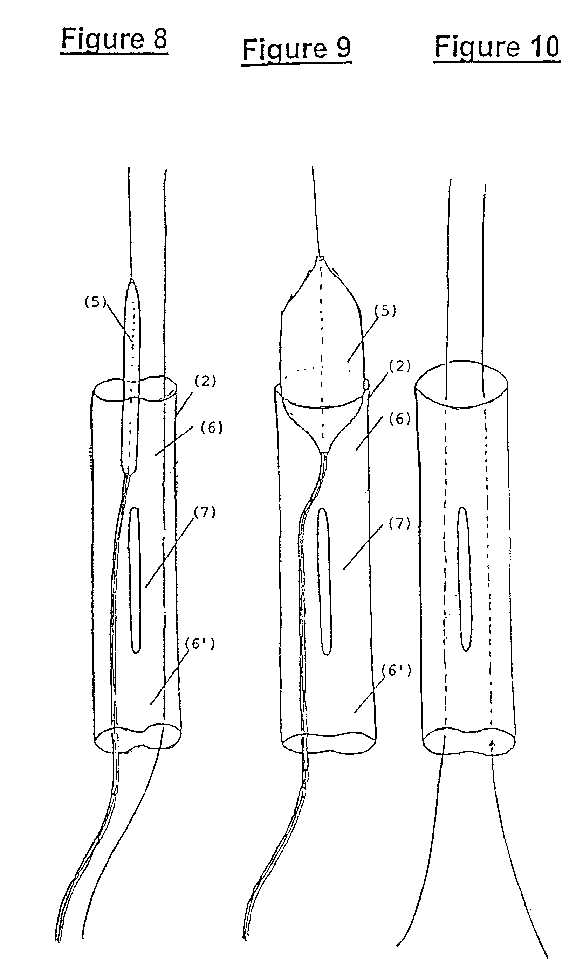

Les figures 8 à 10 illustrent l'ouverture de la partie supérieure de l'extrémité tubulaire proximale (6) du corps prothétique principal bifide (2) à l'aide du ballonnet (5).

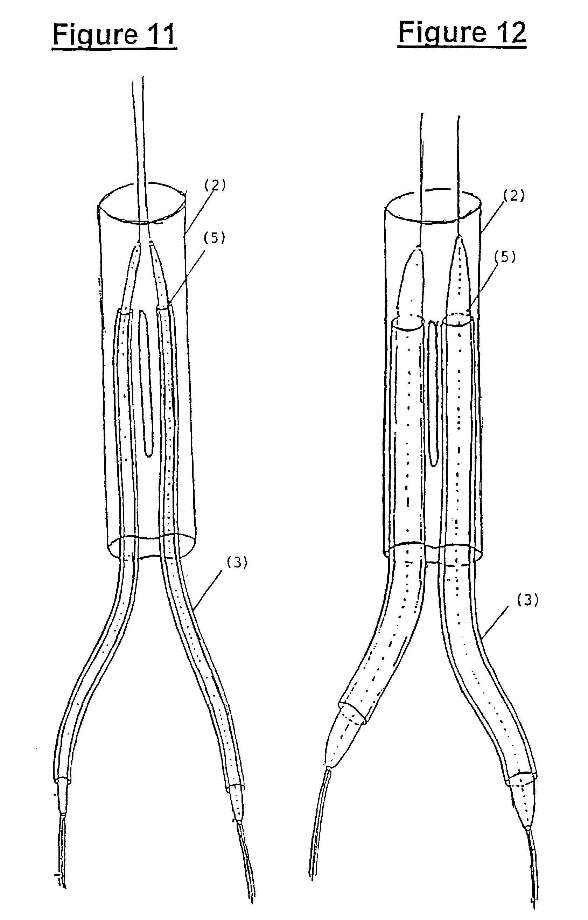

Les figures 11 et 12 montrent l'ascension et l'ouverture des branches prothétiques de bifurcation (3) dans le corps prothétique principal bifide (2) à l'aide des ballonnets (5).

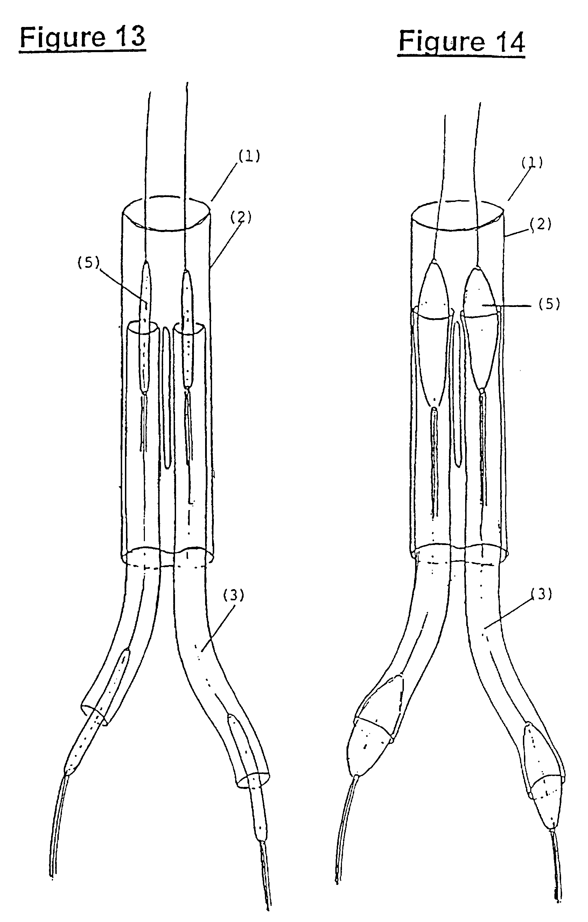

Les figures 13 et 14 montrent la possibilité de surdilater des points d'ancrage supérieur et inférieur des branches prothétiques de bifurcation (3).

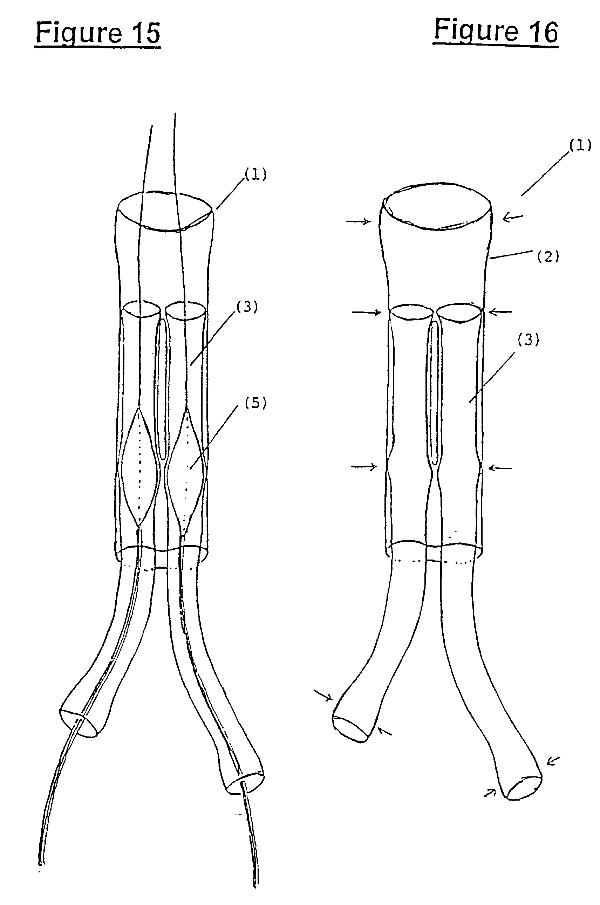

La figure 15 montre la possibilité de surdilater les points d'ancrage des branches prothétiques de bifurcation (3) au niveau de la partie supérieure de l'extrémité tubulaire distale (6'), c'est-à-dire au niveau de la division de l'extrémité tubulaire distale (6') en deux branches ou tubes de bifidité (7).

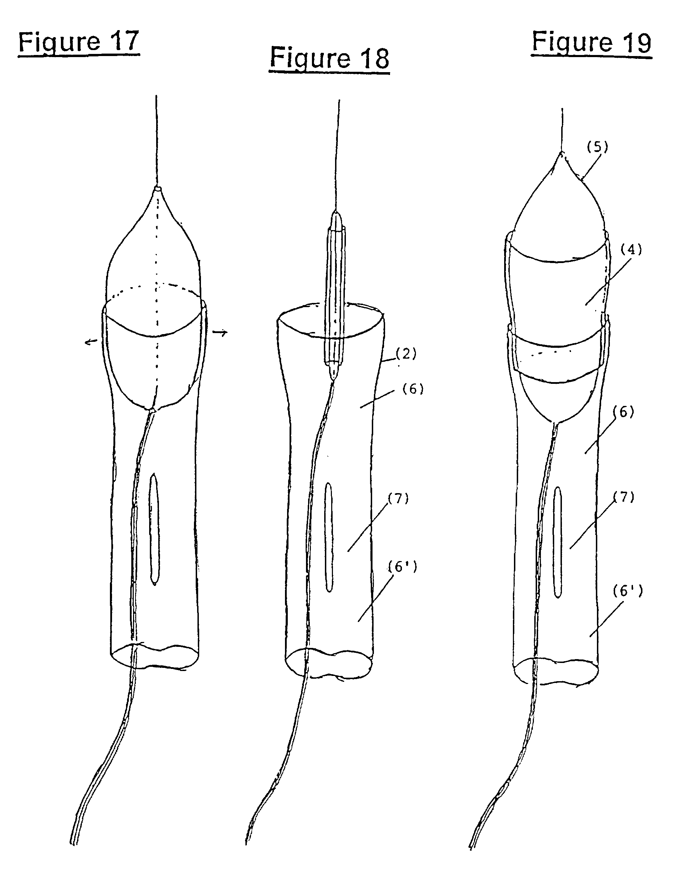

La figure 17 montre la possibilité de surdilater la partie supérieure de l'extrémité tubulaire proximale (6), et les figures 18 et 19 illustrent l'installation d'un étage d'ajustage (4) et sa dilatation par un ballonnet (5). On peut également envisager l'adjonction de plusieurs étages d'ajustage (4) si nécessaire.

La figure 16 montre l'aspect global du corps prothétique bifide (2) surdilaté à la partie supérieure de l'extrémité tubulaire proximale (6), et des branches prothétiques de bifurcation (3) après surdilatage.

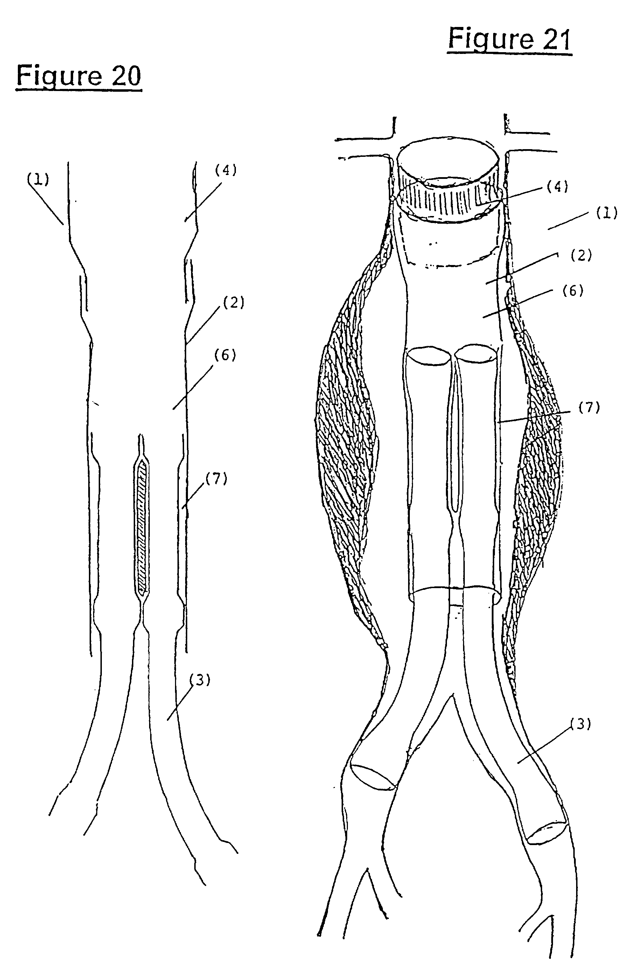

L'expansion forcée des points d'ancrage, représenté dans les figures 13 à 16, permet d'améliorer le « verrouillage » ou blocage des différents éléments de l'endoprothèse et par là la stabilité et l'étanchéité de l'ensemble de l'endoprothèse (1). Ainsi, le corps prothétique bifide (2) repose plus précisément sur les parois de. l'artère à réparer et les branches prothétiques de bifurcation (3) reposent elles-mêmes sur les parois du corps prothétique bifide principal (2), ainsi que sur les parois des artères de bifurcation (cf. figure 21).

La figure 21 montre la correction d'un anévrisme de l'aorte abdominale par la mise en place de l'endoprothèse modulaire bifurquée à corps principal bifide ; l'anévrisme est complètement exclu par l'endoprothèse et la circulation artérielle est maintenue à travers la prothèse.
De cette façon, les parois artérielles sont complètement protégées et la continuité artérielle assurée.

Les endoprothèses (1) selon l'invention trouvent un emploi pour la reconstruction et/ou la recanalisation des segments artériels pathologiques et notamment de ceux qui présentent une bifurcation, comme par exemple la bifurcation aorto-iliaque.

Ils trouvent de ce fait un emploi en chirurgie réparatrice, notamment chez les malades atteints de lésions de l'aorte ou des artères des membres inférieurs.

L'endoprothèse (1) selon l'invention peut être utilisé seule ou en combinaison avec d'autres dispositifs prothétiques.

## Revendications

1. Endoprothèse modulaire et expansible (1) pour la reconstruction d'une bifurcation artérielle, constituée d'un corps prothétique principal (2), de deux branches prothétiques de bifurcation (3) et éventuellement d'un ou de plusieurs étages d'ajustage (4), **caractérisée en ce que** le corps prothétique principal (2) est un corps prothétique bifide en forme de tube ou cylindre unique à ses extrémités proximale et distale dénommées extrémité tubulaire proximale (6) et extrémité tubulaire distale (6'), se dédoublant ou se divisant entre ses extrémités tubulaires proximale et distale en deux conduits séparés et indépendants, dénommés branches ou tubes de bifidité (7).

2. Endoprothèse modulaire et expansible (1) selon la revendication 1, **caractérisée en ce que** les deux branches ou tubes de bifidité (7) présentent une longueur variable et une position variable par rapport aux extrémités tubulaires uniques proximale (6) et distale (6') du corps prothétique principal (2).

3. Endoprothèse modulaire et expansible (1) selon la revendication 1 et la revendication 2, **caractérisée en ce que** les deux branches ou tubes de bifidité (7) se situent dans la partie médiane du corps prothétique principal (2).

4. Endoprothèse modulaire et expansible (1) selon la revendication 1 et la revendication 2, **caractérisée en ce que** les deux branches ou tubes de bifidité (7) se situent plus près de l'extrémité tubulaire distale (6') du corps prothétique principal (2) que de l'extrémité tubulaire proximale (6).

5. Endoprothèse modulaire et expansible (1) selon la revendication 1 et la revendication 2, **caractérisée en ce que** les deux branches ou tubes de bifidité (7) se situent plus près de l'extrémité tubulaire proximale (6) du corps prothétique principal (2) que de l'extrémité tubulaire distale (6').

6. Endoprothèse modulaire et expansible (1) selon l'une des revendications 1 à 5, **caractérisée en ce que** le rapport entre le diamètre d'une branche ou tube de bifidité (7) et le diamètre de l'extrémité tubulaire unique proximale (6) ou distale (6') est égal à 0,5.

7. Endoprothèse modulaire et expansible (1) selon l'une des revendications 1 à 5, **caractérisée en ce que** le rapport entre le diamètre d'une branche ou tube de bifidité (7) et le diamètre de l'extrémité tubulaire unique proximale (6) ou distale (6') est supérieur à 0,5.

8. Endoprothèse modulaire et expansible (1) selon l'une des revendications 1 à 5, **caractérisée en ce que** le rapport entre le diamètre d'une branche ou tube de bifidité (7) et le diamètre de l'extrémité tubulaire unique proximale (6) ou distale (6') est inférieur à 0,5.

9. Endoprothèse modulaire et expansible (1) selon l'une des revendications 1 à 8, **caractérisée en ce que** les diamètres respectifs des deux branches ou tubes de bifidité (7) sont identiques ou différents.

10. Endoprothèse modulaire et expansible (1) selon l'une des revendications 1 à 9, **caractérisée en ce que** les diamètres respectifs des deux extrémités tubulaires proximale (6) et distale (6') sont identiques ou différents.

11. Endoprothèse modulaire et expansible (1) selon l'une des revendications 1 à 10, **caractérisée en ce que** le diamètre de l'extrémité tubulaire proximale (6) est calculé de façon à occuper toute la lumière artérielle.

12. Endoprothèse modulaire et expansible (1) selon la revendication 1, **caractérisée en ce que** le corps prothétique principal bifide (2) présente une longueur comprise entre 1 et 20 cm, et de préférence entre 8 et 12 cm.

13. Endoprothèse modulaire et expansible (1) selon la revendication 1 ou la revendication 10, **caractérisée en ce que** le diamètre des extrémités tubulaires proximale (6) et distale (6') est compris entre 2 et 35 mm, et de préférence entre 12 et 25 mm.

14. Endoprothèse modulaire et expansible (1) selon la revendication 1 ou la revendication 2, **caractérisée en ce que** les deux branches ou tubes de bifidité (7) présentent une longueur comprise entre 0,1 et 18 cm, et de préférence entre 1 et 5 cm.

15. Endoprothèse modulaire et expansible (1) selon la revendication 1 ou la revendication 8, **caractérisée en ce que** le diamètre des deux branches ou tubes de bifidité (7) est compris entre 0,1 et 20 mm, et de préférence entre 7 et 12 mm.

16. Endoprothèse modulaire et expansible (1) selon la revendication 1, **caractérisée en ce que** chacune des deux branches prothétiques de bifurcation (3) est disposée séparément à l'intérieur des deux branches ou tubes de bifidité (7) du corps prothétique principal bifide (2).

17. Endoprothèse modulaire et expansible (1) selon la revendication 1 ou la revendication 16, **caractérisée en ce que** les deux branches prothétiques de bifurcation (3) peuvent être disposées à des niveaux différents dans chacune des deux branches ou tubes de bifidité (7) du corps prothétique principal bifide (2) en faisant varier le niveau d'ancrage de chaque branche prothétique de bifurcation (3) dans la branche ou tube de bifidité correspondant (7).

18. Endoprothèse modulaire et expansible (1) selon la revendication 1, **caractérisée en ce que** l'on ajoute un ou plusieurs étages d'ajustage (4) sur la partie supérieure de l'extrémité tubulaire proximale (6) du corps prothétique principal bifide (2) pour atteindre le niveau recherché dans l'artère.

## Claims

1. Modular and expansible endoprosthesis (1) for the rebuilding of an arterial branching consisting of a main prosthetic body (2), of two prosthetic branches of branching (3) and optionally of one or several stages of adjustment (4) wherein the main prosthetic body (2) is a prosthetic bifid body in a single tubular or cylindrical form at its proximal and distal ends, named proximal tubular and (6) and distal tubular end (6') which disdouble or partition between its proximal and distal tubular ends into two distinct and independent ducts, branches or bifidity tubes (7).

2. Modular and expansible endoprosthesis (1) according to claim 1, wherein the two branches or bifidity tubes (7) show a variable length and a variable position as regard to the single proximal (6) and distal (6') tubular ends of the main prosthetic body (2).

3. Modular and expansible endoprosthesis (1) according to claim 1 and 2, wherein the two branches or bifidity tubes (7) are located in the middle part of the main prosthetic body (2).

4. Modular and expansible endoprosthesis (1) according to claim 1 and claim 2, wherein the two branches or bifidity tubes (7) are located more close of the distal tubular end (6') of the main prosthetic body (2) than of the proximal tubular end (6).

5. Modular and expansible endoprosthesis (1) according to claim 1 and claim 2 wherein the two branches or bifidity tubes (7) are located more close of the proximal tubular end (6) of the main prosthetic body (2) than of the distal tubular end (6').

6. Modular and expansible endoprosthesis (1) according to one of claims 1 to 5, wherein the ratio between the diameters of a single proximal or distal tubular end (6) or (6') is equal to 0,5.

7. Modular and expansible endoprosthesis (1) according to one of claims 1 to 5, wherein the ratio between the diameter of a branch or bifidity tube (7) and the diameter of the single proximal or distal tubular end (6) or (6') is higher than 0,5.

8. Modular and expansible endoprosthesis (1) according to one of the claims 1 to 5, wherein the ratio between the diameter of a branch or bifidity tube (7) and the diameter of the single proximal and distal tubular end (6) or (6') is lower than 0.5.

9. Modular and expansible endoprosthesis (1) according to one of the claims 1 to 8, wherein the respective diameters of the two branches or bifidity tube (7) are the same or different.

10. Modular and expansible endoprosthesis (1) according to one of the claims 1 to 9, wherein the respective diameters of the two proximal and distal tubular end (6) and (6') are the, same or different.

11. Modular and expansible endoprosthesis (1) according to one of the claims 1 to 10, wherein the diameter of the proximal tubular end (6) is so calculated that it occupies the whole arterial lumen.

12. Modular and expansible endoprosthesis (1) according to claim 1, wherein the bifid main prosthetic body (2) shows a length ranging between 1 and 20 cm and preferably between 8 and 12 cm.

13. Modular and expansible endoprosthesis (1) according to claim 1 or claim 10, wherein the diameter of the proximal end distal tubular ends (6) and (6') ranges between 2 and 35 mm and preferably between 12 and 25 mm.

14. Modular and expansible endoprosthesis (1) according to claim 1 or claim 2, wherein the two branches or bifidity tube (7) show a lenghth ranging between 0,1 and 18 cm and preferably between 1 and 5 cm.

15. Modular and expansible endoprosthesis (1) according to claim 1 or claim 8, wherein the diameter of the two branches of bifidity tube (7) ranges between 0,1 and 20 mm and preferably between 7 and 12 mm.

16. Modular and expansible endoprosthesis (1) according to claim 1, wherein each of the prosthetic branch of branching (3) are separately disposed inside the two branches or bifidity tube (7) of the main bifid prosthetic body (2).

17. Modular and expansible endoprosthesis (1) according to claim 1 or claim 16, wherein the two prosthetic branches of branching (3) may be disposed at different levels in each of both branches or bifidity tube (7) of the main bifid prosthetic body (2) by varying the anchoring level of each prosthetic branch of branching (3) into the corresponding branch or bifidity tube (17).

18. Modular and expansible endoprosthesis (1) according to claim 1, wherein one adds one or several stages of adjustment (4) on the upper part of the proximal tubular end (6) of the main bifid prosthetic body (2) to reach the desired level in the artery.

## Patentansprüche

1. Modulare und ausdehnbare Endoprosthesis (1) für den Wiederaufbau einer arteriellen Abzweigung aus einem prosthetichen Hauptboden (2), aus zwei prostethischen Abzweigungsasten (3) und gegebenenfalls einen oder mehreren Justierungsstufen (4) dardurch **gekennzeichnet** daß das prostethische Hauptboden (2) ein in zwei gleichen Teile geteilt als eine einzige Rohr oder Zylinder an seiner proximale rohrenförmige Ende (6) und als distale rohrenförmige Ende (6') genannt wird, die sich halbiert oder verteilt zwischen seiner proximalen und distalen rohrenförmige Ende in zwei getrennte und unabhängige Leitungen, die so-genannte Aste oder Bifidierungsrohre (7)

2. Modulare und ausdehnbare Endoprosthesis (1) nach Anspruch 1, **dadurch gekennzeichnet daß** die zwei Zweige oder Bifidierungsrohre (7) eine wechselnde Länge und eine wechselnde Stellug zeigen, im Zusammenhang mit der einzige rohrenförmige proximale und distale Ende des prothetischen Hauptboden (2).

3. Modulare und ausdehnbare Endoprosthesis (1) nach Anspruch 1 und Anspruch 2, **dadurch gekennzeichnet daß** die zwei Zweige oder Bifidierungsrohre (7) in der mittlere Teile des prosthetischen Hauptbodens (2) liegen.

4. Modulare und ausdehnbare Endoprosthesis (1) nach Anspruch 1 und Anspruch 2, **dadurch gekennzeichnet daß** die zwei Zweige oder Bifidierungsrohre (7) nähere die distale rohrenförmige Ende (6') der prosthetischen Hauptboden (2) als die proximale rohrenförmige Ende (6) liegen.

5. Modulare und ausdehnbare Endoprosthesis (1) nach Anspruch 1 und Anspruch 2, **dadurch gekennzeichnet daß** die zwei Zweige oder Bifidierungsrohre (7) nähere die proximale rohrenförmige Ende (6') der prosthetischen Hauptboden (2) als die distale rohrenförmige Ende (6'), liegen.

6. Modulare und ausdehnbare Endoprosthesis (1) nach Anspruch 1bis 5, **dadurch gekennzeichnet daß** das Verhältnis des Durchmessers einer Zweige oder Bifidierungsrohre (7) zu dem Durchmesser der rohrenförmige einzige proximale (6) oder distale (6') Ende, gleich 0,5 ist.

7. Modulare und ausdehnbare Endoprosthesis (1) nach Anspruch 1bis 5, **dadurch gekennzeichnet daß** das Verhältnis des Durchmessers einer Zweige oder Bifidierungsrohre (7) zu dem Durchmesser der einzige proximale (6) rohrenförmige Ende höher als 0,5 liegt.

8. Modulare und ausdehnbare Endoprosthesis (1) nach Anspruch lbis 5, **dadurch gekennzeichnet daß** das Verhältnis des Durchmessers einer Zweig oder Bifidierungsrohre (7) zu dem Durchmesser der einzige proximale (6) oder distale (6') rohrenförmige Ende, nieder als 0,5 liegt.

9. Modulare und ausdehnbare Endoprosthesis (1) nach Anspruch lbis 8, **dadurch gekennzeichnet daß** die jeweilige Durchmesser der zwei Zweige oder Bifidierungsrohre (7) gleich oder verschieden sind.

10. Modulare und ausdehnbare Endoprosthesis (1) nach Anspruch 1bis 9, **dadurch gekennzeichnet daß** die jeweilige Durchmesser der zwei rohrenförmige proximale oder distale Ende gleich oder verschieden sind.

11. Modulare und ausdehnbare Endoprosthesis (1) nach Anspruch 1 bis 10, **dadurch gekennzeichnet daß** der Durchmesser der proximale rohrenförmige Ende (6) ist so berechnet daß er die ganze arterielle Öffnung besetzt.

12. Modulare und ausdehnbare Endoprosthesis (1) nach Auspruch 1 **dadurch gekennzeichnet daß** das bifid prosthetischen Hauptboden (2) eine zwischen 1 und 20 cm eingeschlossene Länge und vorzugsweise zwischen 8 und 12 cm, aufweist.

13. Modulare und ausdehnbare Endoprosthesis (1) nach Anspruch 1 oder Anspruch 10, **dadurch gekennzeichnet daß** der Durchmesser der proximale (6) und distale rohrenförmige Ende, zwischen 2 und 35 mm eingeschlossen wird, vorzugsweise zwischen 12 und 25 mm.

14. Modulare und ausdehnbare Endoprosthesis (1) nach Anspruch 1 oder Anspruch 8, **dadurch gekennzeichnet daß** die zwei Zweige oder Bifidierungsrohre (7) eine zwischen 0,1 und 18 cm liegende Länge aufweisen.

15. Modulare und ausdehnbare Endoprosthesis (1) nach Anspruch 1 oder Anspruch 8, **dadurch gekennzeichnet daß**, der Durchmesser der zwei Zweige -oder Bifidierungsrohre- (7) zwischen 0,1 und 20 mm liegt und vorzugsweise zwischen 7 und 12 mm.

16. Modulare und ausdehnbare Endoprosthesis (1) nach Anspruch 1, **dadurch gekennzeichnet daß** jede prosthetische Zweige von Abzweigung (3) separate innerhalb der zwei Zweige oder Bifidierungsrohre (7) des prosthetischen bifiden Hauptbodens (2) angeordnet wird.

17. Modulare und ausdehnbare Endoprosthesis (1) nach Anspruch 1 oder Anspruch 16, **dadurch gekennzeichnet daß**, die zwei prosthetische Zweige von Abzweigung (3) am verschieden Hohen in jede der zwei Zweige -oder Bifidierungsrohre (7) des bifiden prosthetischen Hauptbodens (2) durch Variierung der Verankerungshöhe der jeweilige prosthetische Zweige für Abzweigung (3) in der jeweilige Zweige oder Bifidierungsrohre (7) angeordnet werden können.

18. Modulare und ausdehnbare Endoprosthesis (1) nach Anspruch 1 **dadurch gekennzeichnet daß**, einen oder mehreren Justierungsstufen (4) zu dem oberen Teil von der proximale röhrenförmige Ende (6) des bifiden prosthetischen Hauptbodens (2) so versetzt werden daß der gewunschte Höhe in der Ader erreicht wird.
